# EUROPEAN PATENT SPECIFICATION

(11) **EP 3 990 065 B1**
(45) Date of publication and mention of the grant of the patent: **25.12.2024**
(21) Application number: 20743242.8
(22) Date of filing: 26.05.2020
(51) Int. Cl.: A61M 11/00

(54) **RATCHET MECHANISM**
RATSCHENMECHANISMUS
MÉCANISME DE ROCHET

(30) Priority: 01.07.2019 GB 201909476
(43) Date of publication of application: 04.05.2022
(73) Proprietor: Merxin Ltd, King's Lynn, Norfolk PE30 5BY (GB)
(72) Inventor: STUART, Adam, East Leake, Loughborough LE126JA (GB)
(74) Representative: Basck Limited
(86) International application number: PCT/IB2020/054960
(87) International publication number: WO 2021/001705

(56) References cited:
- WO-A2-2004/026380
- DE-A1- 102011 088 252
- FR-A- 577 156

## Description

### TECHNICAL FIELD

The present disclosure relates generally to a ratchet mechanism for drug inhalers to assist a patient with loading of dosage therein.

### BACKGROUND

Generally, several medicines are provided in fluid form, such as a solution or suspension of particles in a propellant or aqueous medium, and are adapted for oral inhalation by a patient. The oral inhalation medicines are typically used for treating patients suffering from respiratory diseases, lung diseases and so forth. For such medication to be delivered from aqueous medium, the fluids may be forced under pressure through micro-formed nozzles in a slow-moving mist. The force needed may exceed that which some patients are able to administer at a time of immediate need, so such inhalers typically operate by a twisting action that is separate from the inhalation manoeuvre. Furthermore, when the force has to be applied to prime the inhaler ready for use, the patient may not complete the priming manoeuvre in one go.

There are several problems associated with such inhalers. One such problem is that a high value of torque is required for loading the inhaler to attain a fully loaded state, which may be impossible for patients with limited strength or dexterity. Furthermore, if the inhaler is released before attaining the fully loaded state, the inhaler is activated in a pre-mature state and a partial dose is released. The partial dose that is released is wasted and may not be registered by a counter mechanism leading to an undercount of the released doses. This may ultimately lead to a situation where the user believes that there are still remaining doses in an empty inhaler, which is not an acceptable situation.

Therefore, in light of the foregoing discussion, there exists a need to overcome the aforementioned drawbacks with the conventional drug inhalers. WO9114468 describes an inhaler for delivering a metered quantity of fluid using a lever or cam mechanism.

### SUMMARY

The present disclosure seeks to provide a ratchet mechanism. The present disclosure seeks to provide a solution to the existing problem of high loading forces of the portable inhalers required for loading the portable inhalers. Furthermore, embodiments of the present disclosure also seek to provide a solution to the existing problem of delivering a partial dose due to a failure of complete loading of the portable inhaler. An aim of the present disclosure is to provide a solution that overcomes at least partially the problems encountered in prior art, and provides a resilient, robust ratchet mechanism which can robustly hold back the high loading torque of a soft mist inhaler allowing the patient to pause the twist action required to load the drug inhaler.

In an aspect, an embodiment of the present disclosure provides a ratchet mechanism comprising:
- a ratchet ring rotatable about an axis relative to a chassis, with a general direction of rotation determined by teeth of the ratchet mechanism, wherein the ratchet ring has a first set of teeth arranged regularly around the ratchet ring and protruding in a first axial direction from the ratchet ring and a second set of teeth arranged regularly around the ratchet ring and protruding in a second opposite axial direction from the ratchet ring; and
- the chassis which has a third set of teeth arranged regularly and protruding in the second axial direction and generally opposed to the first set of teeth, and a fourth set of teeth arranged regularly and protruding in the first axial direction and generally opposed to the second set of teeth,
wherein:
- each tooth protruding in the first axial direction or the second axial direction has a leading edge and a trailing edge with respect to the general direction of rotation of the ratchet ring,
- the trailing edge on each tooth of the first set of the teeth and the second set of teeth is oriented in the axial direction of protrusion and the leading edge on each tooth of the first set of teeth and the second set of teeth is angled towards the trailing direction with respect to the axial direction of protrusion, and
- the leading edge on each tooth of the third set of teeth and the fourth set of teeth is oriented in the axial direction of protrusion and the trailing edge on each tooth of the third set of teeth and the fourth set of teeth is angled towards the leading direction with respect to the axial direction of protrusion.

Optionally, the teeth from the first set of teeth and the third set of teeth are arranged to align with each other at different relative position of the ratchet ring and the chassis to those at which teeth from the second set of teeth and the fourth set of teeth are arranged to align.

Optionally, the teeth are arranged such that opposition of teeth from the first set of teeth and the third set of teeth occurs at primary positions of the ratchet ring and opposition of teeth from the second set of teeth and the fourth set of teeth occurs at secondary positions of the ratchet ring, such that the primary positions and the secondary positions alternate as the ratchet ring is rotated in the general direction of rotation.

Optionally, the spacing between distal ends of the third set of teeth and the fourth set of teeth is less than the spacing between distal ends of the first set of teeth and second set of teeth.

Optionally, the spacing between distal ends of the third set of teeth and the fourth set of teeth is greater than an axial depth of the ratchet ring.

Optionally, the first set of teeth and the second set of teeth protrude from opposite surfaces of the ratchet ring.

Optionally, the number of teeth in the first set of teeth and the number of teeth in the third set of teeth are related by an integer multiple.

Optionally, the number of teeth in the second set of teeth and the number of teeth in the fourth set of teeth are related by an integer multiple.

Optionally, the number of teeth in the first set of teeth is equal to the number of teeth in the second set of teeth.

In another aspect, the present disclosure provides a portable inhaler comprising a ratchet mechanism of the present disclosure, which comprises a rotary actuation mechanism having a driving component coupled to rotary movement, by a user, of a first part of the portable inhaler relative to a second part of the portable inhaler.

Optionally, the ratchet ring and a driving component of the rotary actuation mechanism have mutually interfering ribs or slots such that the ratchet ring and the driving component, e.g. a cage component, are rotationally coupled.

Optionally, the third set of teeth and the fourth set of teeth extend from an inner wall of the second part of the portable inhaler, e.g. the chassis.

Embodiments of the present disclosure substantially eliminate or at least partially address the aforementioned problems in the prior art, and enable the user to incrementally load the portable inhaler by pausing the twist action as and when required by the user.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:
- FIG. 1: is a schematic illustration of a portable inhaler having a rotary actuation mechanism, in accordance with an embodiment of the present disclosure;
- FIG. 2: is a schematic illustration of a ratchet mechanism, in accordance with an embodiment of the present disclosure;
- FIG. 3A: is a schematic illustration of a ratchet mechanism in a primary position, in accordance with an embodiment of the present disclosure;
- FIG. 3B: is a schematic illustration of a ratchet mechanism in a secondary position, in accordance with an embodiment of the present disclosure;
- FIG. 4A: is a schematic illustration of a ratchet mechanism in a primary position, in accordance with another embodiment of the present disclosure;
- FIG. 4B: is a schematic illustration of a ratchet mechanism in a secondary position, in accordance with another embodiment of the present disclosure;
- FIG. 5: is a schematic illustration of a portable inhaler implementing a ratchet mechanism of FIG. 2, in accordance with another embodiment of the present disclosure; and
- FIG. 6: is a schematic illustration of an exploded view of a ratchet mechanism, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In one aspect, an embodiment of the present disclosure provides a ratchet mechanism comprising:
- a ratchet ring rotatable about an axis relative to a chassis, with a general direction of rotation determined by teeth of the ratchet mechanism, wherein the ratchet ring has a first set of teeth arranged regularly around the ratchet ring and protruding in a first axial direction from the ratchet ring and a second set of teeth arranged regularly around the ratchet ring and protruding in a second opposite axial direction from the ratchet ring;
- the chassis having a third set of teeth arranged regularly and protruding in the second axial direction and generally opposed to the first set of teeth, and a fourth set of teeth arranged regularly and protruding in the first axial direction and generally opposed to the second set of teeth,
wherein:
- each tooth protruding in the first axial direction or the second axial direction has a leading edge and a trailing edge with respect to the general direction of rotation of the ratchet ring,
- the trailing edge on each tooth of the first set of the teeth and the second set of teeth is oriented in the axial direction of protrusion and the leading edge on each tooth of the first set of teeth and the second set of teeth is angled towards the trailing direction with respect to the axial direction of protrusion, and
- the leading edge on each tooth of the third set of teeth and the fourth set of teeth is oriented in the axial direction of protrusion and the trailing edge on each tooth of the third set of teeth and the fourth set of teeth is angled towards the leading direction with respect to the axial direction of protrusion.

The present disclosure provides a ratchet mechanism for incrementally loading a portable inhaler in a general direction of rotation by allowing the motion of a ratchet ring in the general direction of rotation and restricting the motion in a direction opposite to the general direction of rotation. The embodiments of the present disclosure provide a robust ratchet mechanism that is operable to resiliently hold back high loading torque of the portable inhaler. The ratchet mechanism allows the user to pause the loading action several times, and thereby incrementally reach the loading state of the portable inhaler. Such a mechanism allows the patient to carry out the loading operation with minimal force, providing the user with a user-friendly portable inhaler. Beneficially, the ratchet mechanism provides complete manual actuation control to the user for loading the inhaler, thereby preventing a pre-mature activation of the inhaler and an unintentional release of the fluid dose therefrom. Furthermore, the ratchet mechanism also prevents an erroneous undercount of a counter in the drug inhaler.

Throughout the present disclosure, the term *"portable inhaler"* as used herein refers to inhalation drug dose dispensers, that deliver a predetermined amount of medication (for example, the portable inhaler may contain asthma medicine such as fluticasone propionate) in a form of spray, which is inhaled by a user (such as a patient). Furthermore, portable inhalers are referred to micro-liquid dispensing systems employed to dispense a fluid in small quantities with increased accuracy. The portable inhalers can control and manipulate small volumes of fluid (generally in micrometric volumes). The portable inhaler includes a driving component, for example a cage component of a rotary actuation mechanism. The driving component and a first part of the inhaler housing which is rotatable relative to another part of the housing are mutually coupled to provide actuation for release of dosage. A second part of the inhaler may be in the form of a chassis, which may comprise one or more parts. The second part of the inhaler suitably is not rotatable relative to a mouthpiece of the inhaler. The portable inhaler operates in conjunction with an actuation system, with the actuation system comprising a housing having a loading end and an open end (mouthpiece). In order to receive a dose from the mouthpiece, the loading end of the inhaler is to be rotated by applying a torque in the direction of rotation. Furthermore, once in a loaded state, a pressure force is exerted to dispense the fluid from the portable inhaler through a narrow outlet (such as a nozzle). Furthermore, the narrow outlet allows the fluid to be delivered with a higher pressure. The portable inhaler includes a container (which contains the fluid) that is detachably attached inside the inhaler, and may be detached when not in use. Notably, a metered amount of dose is drawn from the container, and delivered through the nozzle via the mouthpiece. Moreover, the portable inhalers mimic a shape of a cylinder, and has a length that could easily be held in a palm of the user.

The ratchet ring is rotatable about an axis, with a general direction of rotation determined by teeth of the ratchet mechanism. The ratchet ring has a first set of teeth arranged regularly around the ratchet ring and protruding in a first axial direction from the ratchet ring, and a second set of teeth arranged regularly around the ratchet ring and protruding in a second opposite axial direction from the ratchet ring. The term *"ratchet ring"* as used herein refers to an annular ring having an axial depth, and having two peripheral surfaces (namely, a first peripheral surface and a second peripheral surface) spaced apart by the axial depth of the ratchet ring. The axial depth may be defined as the distance between the first peripheral surface and the second peripheral surface of the ratchet ring. The first peripheral surface and the second peripheral surface are notably opposite to each other.

The first set of teeth are arranged on the first peripheral surface of the ratchet ring, and the second set of teeth are arranged on the second peripheral surface of the ratchet ring. The first set of teeth are arranged uniformly on the first peripheral surface of the ratchet ring, i.e. the teeth in the first set of teeth are equidistant from each other. Similarly, the second set of teeth are arranged uniformly on the second peripheral surface of the ratchet ring, i.e. the teeth in the second set of teeth are equidistant from each other. Optionally, the first set of teeth and the second set of teeth may be arranged non-uniformly on the ratchet ring, i.e. the teeth in the respective first set of teeth or the second set of teeth are not arranged to be equidistant from each other. Further, as aforementioned, the first set of teeth protrudes in a first axial direction from the ratchet ring. Herein, the *"first axial direction"* refers to a direction parallel to the central axis (an axis passing through the center) of the ratchet ring and towards the mouthpiece of the portable inhaler. Furthermore, the second set of teeth protrudes in a second axial direction from the ratchet ring. Herein, the *"second axial direction"* refers to a direction parallel to the central axis of the ratchet ring and away from the mouthpiece of the portable inhaler. It will be appreciated that the first axial direction and the second axial direction are co-axial and opposite to each other. Herein, the first axial direction and the second axial direction are merely indicative of two opposite sense of direction about the axis of rotation of the ratchet ring, and should not unduly limit the scope of the claims appended herein. Notably, the ratchet ring is configured to be rotated about the central axis, and the general direction of rotation is determined by teeth of the ratchet mechanism, which has been discussed in detail later in the description.

Further, the ratchet mechanism comprises a chassis. Herein the term *"chassis"* refers to a body of the portable inhaler. The chassis encompasses the entire body of the portable inhaler, and may comprise one or more parts, fixed together. Notably, the chassis remains stationary when the portable inhaler is subjected to a rotational motion. Furthermore, the chassis may provide the central axis, about which the ratchet ring is configured to be rotated. It will be appreciated that a shape of the chassis is akin to a shape of the internal structure of the portable inhaler. In an example, the driving component and the cage component are dominantly cylindrical in shape, therefore the chassis is also cylindrical in shape.

The chassis has a third set of teeth arranged regularly and protruding in the second axial direction and generally opposed to the first set of teeth. Optionally, the third set of teeth protrudes from an inner wall of the chassis of the portable inhaler. The third set of teeth are arranged in a manner so as to engage with the first set of teeth in order to restrict the motion in a direction opposite to the general direction of rotation of the driving component and the ratchet ring coupled thereto. The third set of teeth are arranged uniformly on the inner wall of the chassis i.e. the teeth in the third set of teeth are equidistant from each other. Optionally, the third set of teeth may be arranged non-uniformly on the inner wall, i.e. the teeth in the third set of teeth are not arranged to be equidistant from each other. Further, the chassis has a fourth set of teeth arranged regularly and protruding in the first axial direction and generally opposed to the second set of teeth. Optionally, the fourth set of teeth protrudes from an inner wall of the chassis of the portable inhaler. The fourth set of teeth are arranged in a manner so as to engage with the second set of teeth in order to restrict the motion in a direction opposite to the general direction of rotation of the driving component and the ratchet ring coupled thereto. The fourth set of teeth are arranged uniformly on the chassis i.e. the teeth in the fourth set of teeth are equidistant from each other. Optionally, the fourth set of teeth may be arranged non-uniformly on the inner wall, i.e. the teeth in the fourth set of teeth are not arranged to be equidistant from each other.

Optionally, the fourth set of teeth are arranged on a counter mechanism. The counter mechanism may be in the form of an annular ring, and having the fourth set of teeth protruding from an upper surface thereof. The counter mechanism is capable of determining a number of used doses and display a number of remaining doses on a display counter. Herein, the fourth set of teeth oppose the movement of ratchet in an opposite direction and efficiently register a number of used doses at the same time.

Optionally, the ratchet ring and the driving component of the rotary actuation mechanism have mutually interfering ribs or slots such that the ratchet ring and the cage component are rotationally coupled. The ratchet ring is placed in a concentric manner with respect to the driving component and the cage component. In particular, the ratchet ring is rotationally coupled to the cage component of the portable inhaler. Throughout the present disclosure, the term *"cage component"* refers to a cylindrical structure that Notably, the ratchet ring may be rotationally coupled to the outer surface of the cage component, such that the ratchet ring moves with a rotational movement of the cage component. It will be appreciated that in such a case, the first set of teeth and the second set of teeth are also moving as the ratchet ring rotates, whereas the third set of teeth and the fourth set of teeth are stationary. In an alternate embodiment, the ratchet ring having the first set of teeth and the second set of teeth may be coupled to the chassis, and the third set of teeth and the fourth set of teeth protrude from the cage component to achieve the same functioning, without departing from the scope of the present disclosure. In such a case, the first set of teeth and the second set of teeth are stationary, whereas the third set of teeth and the fourth set of teeth are moving, as the portable inhaler is rotated in the direction of rotation.

Optionally, the spacing between distal ends of the third set of teeth and the fourth set of teeth is less than the spacing between distal ends of the first set of teeth and the second set of teeth. Hereinafter, the term "distal ends" refers to the tip of each tooth of the corresponding first set of teeth, second set of teeth, third set of teeth and fourth set of teeth. The spacing between two distal ends is determined as the axial distance between the tip of one tooth to the tip of another tooth, protruding in two different axial directions. Notably, the axial distance between the distal ends of the third set of teeth and the fourth set of teeth is less than the axial distance between the distal ends of the first set of teeth and the second set of teeth. Henceforth, the interaction of the first set of teeth with the third set of teeth, and the interaction of the second set of teeth with the fourth set of teeth is made possible in the direction of rotation. Furthermore, the engagement of the first set of teeth with the third set of teeth, and the engagement of the second set of teeth with the fourth set of teeth is made possible in the opposite direction. Optionally, the spacing between the distal ends of the third set of teeth and the fourth set of teeth is greater than the axial depth of the ratchet ring. It will be appreciated that the axial distance between the distal ends of the third set of teeth and the fourth set of teeth is designed to be greater than the axial depth of the ratchet ring, such that the ratchet ring is loosely fitted between the third set of teeth and the fourth set of teeth, allowing the rotation of the ratchet ring in general direction of rotation, and opposing the rotation of the ratchet ring in the opposite direction.

Optionally, the number of teeth in the first set of teeth is equal to the number of teeth in the second set of teeth. The number of teeth on the two opposite surfaces (the first surface and the second surface) of the ratchet ring are equal, such that the backlash arising in the ratchet mechanism is equal at both the primary positions and the secondary positions of the ratchet ring. Optionally, the number of teeth in the first set of teeth and the number of teeth in the third set of teeth are related by an integer multiple. In an example, there are 8 number of teeth in the third set of teeth that extend regularly from the inner wall of the portable inhaler in a circumferential manner. Each tooth is arranged on the inner wall such that each of the teeth are spaced apart by an angle of 45 degrees, thereby making the arrangement of teeth uniform. Further, the number of teeth in the first set of teeth may be an integral multiple of two, thereby the first set of teeth may have 16 number of teeth arranged regularly on the periphery of the first surface, spaced apart by an angle of 27.5 degrees. Notably, the number of teeth in the first set of teeth are twice to that of the number of teeth in the second set of teeth. Therefore, when the ratchet ring is in the primary position there will be two teeth of the first set teeth located between two teeth of the third set of teeth to restrict the rotational motion in the opposite direction. In another example, the number of teeth in the first set of teeth is equal to the number of teeth in the second set of teeth. In such a case, each tooth of the first set of teeth is engaged with one of the teeth of the second set of teeth to restrict the rotational motion in the opposite direction. It will be appreciated that the primary and secondary positions are equivalent to each other in function, and the ratchet mechanism passes these positions alternately when operating in the general direction of rotation. Furthermore, the ratchet ring undergoes vertical displacements as the various sets of teeth ride over each other, which may cause the rotational axis of the ratchet ring to deviate temporarily. However, the overall direction of rotation is referred to as the *"general direction of rotation".*

Optionally, the number of teeth in the second set of teeth and the number of teeth in the fourth set of teeth are related by an integer multiple. In an example, there are 8 number of teeth in the fourth set of teeth that extend regularly from the inner wall of the portable inhaler in a circumferential manner. Each tooth is arranged on the inner wall such that the teeth are spaced apart by an angle of 45 degrees, thereby making the arrangement of teeth uniform. Further, the number of teeth in the second set of teeth may be an integral multiple of two, thereby the first set of teeth may have 16 number of teeth arranged regularly on the periphery of the first surface, spaced apart by an angle of 27.5 degrees. Notably, the number of teeth in the second set of teeth are twice to that of the number of teeth in the fourth set of teeth. Therefore, when the ratchet ring is in the secondary position there will be two teeth of the second set teeth located between two teeth of the fourth set of teeth to restrict the rotational motion in the opposite direction. In another example, the number of teeth in the second set of teeth is equal to the number of teeth in the fourth set of teeth. In such a case, each tooth of the second set of teeth is engaged with one of the teeth of the fourth set of teeth to restrict the rotational motion in the opposite direction.

Notably, a number of teeth and the spacing between teeth in each of the first set, second set, third set and fourth set affects the amount of torque to be applied in the direction of rotation in order to load the portable inhaler. It will be appreciated that greater the number of teeth, greater is the amount of torque required to load the portable inhaler. Furthermore, the amount of torque is directly proportional to the amount of friction between the tips of each tooth of the first set of teeth and the third set of teeth, and also the second set of teeth and the fourth set of teeth. Henceforth, greater the amount of friction between the tips of engaged teeth, greater will be the amount of torque required for loading the portable inhaler. Moreover, the amount of torque is directly proportional to the rotational inertia of the ratchet ring.

Each tooth protruding in the first axial direction or the second axial direction has a leading edge and a trailing edge with respect to the general direction of rotation of the ratchet ring. Herein, the general direction of rotation refers to the direction in which the torque is to be applied in order to load the portable inhaler. In an example, the general direction of rotation is in clockwise sense, therefore when the portable inhaler is rotated in the clockwise sense, the teeth on the ratchet ring and the teeth on the chassis engage with each other to allow movement in the clockwise sense and restrict the movement in the anti-clockwise sense. In another example, the general direction of rotation is in anti-clockwise sense, therefore when the portable inhaler is rotated in the anti-clockwise sense, the teeth on the ratchet ring and the teeth on the chassis engage with each other to allow movement in the anti-clockwise sense and restrict the movement in the clockwise sense.

Notably, the leading edge and the trailing edge of each of the tooth are determined with respect to the general direction of rotation of the ratchet ring. The leading edge and the trailing edge of each of the tooth may be axially aligned in a straight line or angled, depending upon the direction of rotation of the portable inhaler. For example, the leading edge or the trailing edge may have an angled profile or vertical profile. The trailing edge on each tooth of the first set of the teeth and the second set of teeth is oriented in the axial direction of protrusion (herein, first axial direction) and the leading edge on each tooth of the first set of teeth and the second set of teeth is angled towards the trailing direction with respect to the axial direction of protrusion (herein, second axial direction). Further, the leading edge on each tooth of the third set of teeth and the fourth set of teeth is oriented in the axial direction of protrusion (herein, first axial direction) and the trailing edge on each tooth of the third set of teeth and the fourth set of teeth is angled towards the leading direction with respect to the axial direction of protrusion (herein, second axial direction). It will be appreciated that for a smooth, low force rotation of the ratchet ring and the portable inhaler, the angled profile (leading edge) of the first set of teeth and/or the second set of teeth will interact with the angled profile (trailing edge) of the third set of teeth and/or the fourth set of teeth in the direction of rotation. Furthermore, the vertical profile (trailing edge) of the first set of teeth and/or the second set of teeth is engaged with the vertical profile (leading edge) of the third set of teeth and/or the fourth set of teeth restricting a recoil movement of the ratchet ring in a direction opposite to the direction of rotation. For the sake of simplicity and clarity, the general direction of rotation of the portable inhaler and thereby the ratchet ring is considered to be anti-clockwise, unless stated otherwise.

When the portable inhaler is rotated in the anti-clockwise sense for loading the portable inhaler, the leading edge of each tooth of the first set of teeth interacts with the trailing edge of each tooth of the third set of teeth. Further, the leading edge of each tooth of the second set of teeth interacts with the trailing edge of each tooth of the fourth set of teeth, henceforth allowing the rotation of the ratchet ring and the portable inhaler in the direction of rotation. However, when the ratchet ring is rotated in a direction opposite to the direction of rotation, the trailing edge of each tooth of the first set of teeth engages with the leading edge of each tooth of the third set of teeth. Further, the trailing edge of each tooth of the third set of teeth engages with the leading edge of each tooth of the fourth set of teeth. Henceforth, restricting the rotation of the ratchet ring and the portable inhaler in a direction opposite to the direction of rotation. Notably, by restricting the motion of the ratchet ring, the movement of the driving component can be controlled as desired by the user.

Beneficially, such a ratchet mechanism enables the user to incrementally load the inhaler, i.e. in a step-wise manner, thereby making the device user-friendly. Optionally, a portable inhaler is required to have a rotational displacement of 180 degrees in order to load the portable inhaler. In an example, the portable inhaler may be rotated to have a rotational displacement of 180 degrees in a single step. In another example, the portable inhaler may be rotated to have a rotational displacement of 180 degrees in two steps, a first rotational displacement of 90 degrees followed by a second rotational displacement of 90 degrees. In another example, the portable inhaler may be rotated to have a rotational displacement of 180 degrees in three steps, a first rotational displacement of 60 degrees followed by a second rotational displacement of 60 degrees followed by a third rotational displacement of 60 degrees. It will be appreciated that a rotational displacement of 180 degrees may be achieved by any number of incremental rotational displacements and any number of pauses therebetween as desired by the user. Notably, the pauses between the stepped rotational displacements are provided by the ratchet mechanism by resiliently holding the ratchet ring and the driving component in position in an intermediary loading state as well.

Optionally, the teeth from the first set of teeth and the third set of teeth are arranged to align with each other at different relative position of the ratchet ring and the chassis, to those at which teeth from the second set of teeth and the fourth set of teeth are arranged to align. Notably, each tooth of the first set of teeth axially aligns with each tooth of the second set of teeth, and each tooth of the third set of teeth axially aligns with each tooth of the fourth set of teeth. The third set of teeth and fourth set of teeth, protruding from the chassis, may be offset from the first set of teeth and the second set of teeth protruding from the ratchet ring by a definite pre-defined angle. In an example, the third set of teeth and fourth set of teeth are offset by an angle of 15 degrees from the first set of teeth and the second set of teeth.

Optionally, the teeth are arranged such that opposition of teeth from the first set of teeth and the third set of teeth occurs at primary positions of the ratchet ring and opposition of teeth from the second set of teeth and the fourth set of teeth occurs at secondary positions of the ratchet ring, such that the primary positions and the secondary positions alternate as the ratchet ring is rotated in the general direction of rotation. Notably, the first set of teeth and the third set of teeth are arranged to be engaged at different positions of the ratchet ring, and the second set of teeth and the fourth set of teeth are arranged to be engaged at different positions of the ratchet ring, defined by the pre-determined offset angle and the position of the ratchet ring at which the torque applied to the portable inhaler is reduced to zero. When the portable inhaler is in the initial state (unloaded state), the first set of teeth of the ratchet ring may be engaged with the third set of teeth of the chassis; the resultant position is considered as the primary position of the ratchet ring. Further, when the portable inhaler is rotated in the general direction of rotation by 15 degrees, the second set of teeth of the ratchet ring and the fourth set of teeth of the chassis are engaged to restrict the motion of the ratchet ring in the opposite direction; the resultant position is considered as the secondary position of the ratchet ring. It will be appreciated that a value of the offset angle determines the maximum degree of backlash offered by the ratchet ring. Notably, a spacing between the distal ends of the teeth in the first set of teeth and the teeth in the third set of teeth, also a spacing between the distal ends of the teeth in the second set of teeth and the teeth in the fourth set of teeth, determines the offset angle and thereby the degree of backlash offered by the ratchet ring. The offset angle can be increased or decreased by increasing or decreasing the spacing between the distal ends of the teeth in the first set of teeth and the teeth in the third set of teeth, and a spacing between the distal ends of the teeth in the second set of teeth and the teeth in the fourth set of teeth.

As mentioned, the primary positions and the secondary positions of the ratchet ring alternate as the ratchet ring is rotated by applying torque in the general direction of rotation of the ratchet ring. Notably, the ratchet mechanism is configured to oppose the rotational motion of the ratchet ring in the opposite direction by either engaging the first set of teeth with the third set of teeth and/or the second set of teeth with the fourth set of teeth, depending upon the position (primary position or the secondary position) of the ratchet ring. It will be appreciated that the ratchet ring opposes the motion of the portable inhaler in the opposite direction by engaging the ratchet ring with one or both of the third set of teeth (i.e. at the primary positions) and the fourth set of teeth (i.e. at the secondary positions), depending upon which of the sets of teeth is encountered first. In an example, when the torque applied to the portable inhaler is reduced to zero during loading, either the first set of teeth of the ratchet ring may be engaged with the third set of teeth, holding the ratchet ring in place in the primary position, or the second set of teeth of the ratchet ring may be engaged with the fourth set of teeth, holding the ratchet ring in place in the secondary position.

Optionally, the ratchet ring is configured to translate in the axial direction and engage with either of the third set of teeth or the fourth set of teeth. The ratchet ring reciprocates with the translatory motion of the driving component, and is thereby translated between primary positions and a secondary position. In such a case, the ratchet ring is rotationally coupled to the driving component, such that the ratchet ring moves in the axial direction in accordance with the axial movement of the driving component. Notably, the ratchet ring moves axially as well as rotates, reciprocating the movement of the driving component. Herein, the primary position of the ratchet ring is the position when the portable inhaler is in an initial state, i.e. the rotational displacement is zero. At such position, the trailing edge of each tooth of the first set of teeth is engaged with the leading edge of one of teeth of the third set of teeth and the driving component is at the highest position. When the portable inhaler is rotated in the direction of rotation (anti-clockwise), the each of the leading edges of teeth of the first set of teeth and the second set of teeth interact with each other, and the ratchet ring moves gradually in an axially downward direction, as the driving component rotates. The ratchet ring translates in an axially downward direction to attain the secondary position. At such position, the trailing edge of each tooth of the second set of teeth is engaged with the leading edge of one of the teeth of the fourth set of teeth, and the driving component is at the lowest position. Once the portable inhaler is activated, back-rotation may be prevented by features of the rotary actuation mechanism.

### DETAILED DESCRIPTION OF THE DRAWINGS

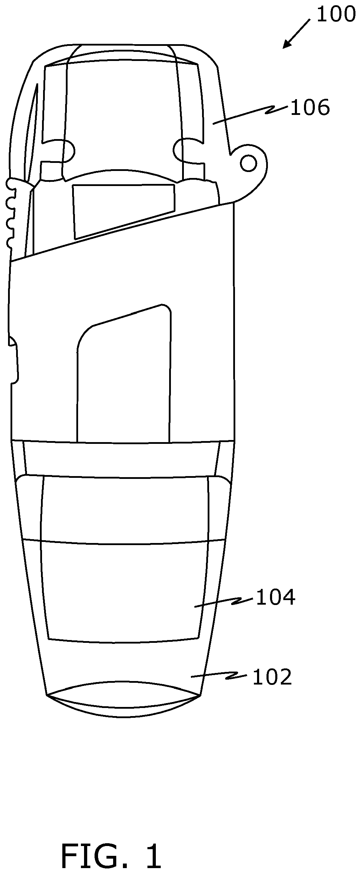

Referring to FIG. 1, illustrated is a schematic illustration of a portable inhaler **100** of the type suitable for including a ratchet mechanism in accordance with an embodiment of the present disclosure. As shown, the portable inhaler **100** comprises a chassis **102** having a loading end **104** and an open end containing a mouthpiece **106.** The chassis **102** encloses a rotary actuation mechanism comprising a driving component (not shown) releasable to dispense a dose as the rotary actuation mechanism translates from a first position to a second position. Further, the driving component is rotationally coupled to a cage component (not shown), and the cage component is rotationally coupled to a ratchet ring (not shown). In order to load the portable inhaler **100,** the loading end **104** is rotated in a counter clockwise direction to provide a rotational displacement of 180 degrees. Herein, the rotational displacement of 180 degrees may be achieved in pauses by incrementally controlling the rotational displacement of the portable inhaler **100** via a ratchet mechanism (not shown).

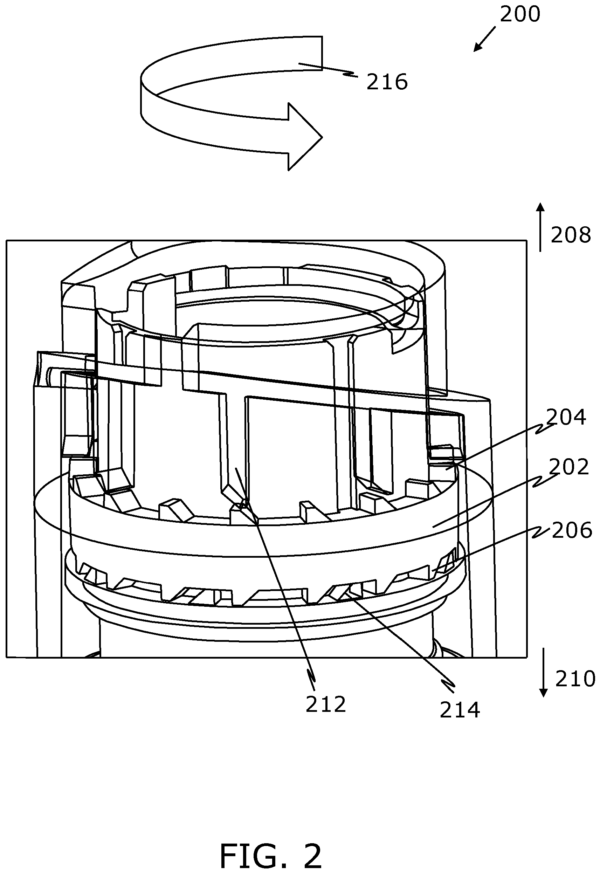

Referring to FIG. 2, illustrated is a schematic representation of a ratchet mechanism **200** for a portable inhaler (such as the portable inhaler of FIG. 1), in accordance with an embodiment of the present disclosure. As shown, the ratchet mechanism **200** comprises a ratchet ring **202** having a first set of teeth **204** and a second set of teeth **206.** Herein, the first set of teeth **204** are arranged regularly around the ratchet ring **202** and protrude in a first axial direction **208** from the ratchet ring **202.** The second set of teeth **206** are arranged regularly around the ratchet ring **202** and protrude in a second axial direction **210** from the ratchet ring **202.** Notably, the first axial direction **208** and the second axial direction **210** are offset by 180 degrees, i.e. the first axial direction **208** and the second axial direction **210** are mutually opposite to each other. Further, the ratchet mechanism **200** comprises a chassis (not shown) having a third set of teeth **212** and a fourth set teeth **214.** The third set of teeth **212** are arranged regularly on an inner wall of the chassis of the portable inhaler and protrude in the second axial direction **210** (opposite to the first set of teeth **204).** The fourth set of teeth **214** are also arranged regularly on the inner wall of the chassis of the portable inhaler and protrude in the first axial direction **208** (opposite to the second set of teeth **206).** Notably, each of the teeth in the first set of teeth **204,** the second set of teeth **206,** the third set of teeth **212,** and the fourth set of teeth **214** have a trailing edge and a leading edge with respect to the direction of rotation **216,** i.e. the leading edge corresponds to the side of each ratchet tooth **204, 206** that leads.

As shown, when the portable inhaler is rotated in the direction of rotation **216** (here, counter clockwise direction) by applying a torque in the direction of rotation, the leading edge of the first set of teeth **204** interact with the leading edge of the third set of teeth **212** and the leading edge of the second set of teeth **206** interacts with the leading edge of the fourth set of teeth **214,** allowing the movement of the ratchet ring in the direction of rotation **216.**

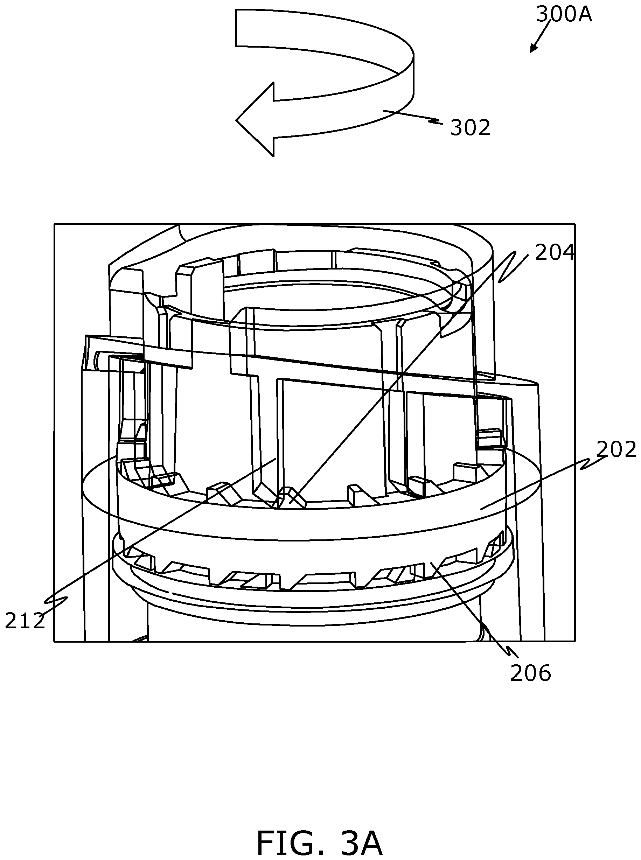

Referring to FIG. 3A, illustrated is a schematic representation a ratchet mechanism **300A** (such as the ratchet mechanism of FIG. 2) in a primary position of the ratchet ring **202,** in accordance with an embodiment of the present disclosure. When the torque applied to the portable inhaler in the direction of rotation (not shown) is reduced to zero prior to achieving a rotational displacement of 180 degrees, the ratchet ring **202** is retracted back in the clockwise direction **302,** under the influence of a spring force which is directed rotationally by inclined features of the rotary actuation mechanism. Furthermore, the retraction of the ratchet ring **202** is opposed by the third set of teeth **212.** The trailing edges of each of the teeth of the first set of teeth **204** engage with the leading edges of the teeth of the third set of teeth **212,** thereby retaining the position of the ratchet ring **202** that was attained by the application of torque in the direction of rotation. This position of the ratchet ring **202** when the first set of teeth **204** engage with the third set of teeth **212** to restrict the movement of the ratchet ring **202** in the direction **302** is the primary position of the ratchet ring **202.**

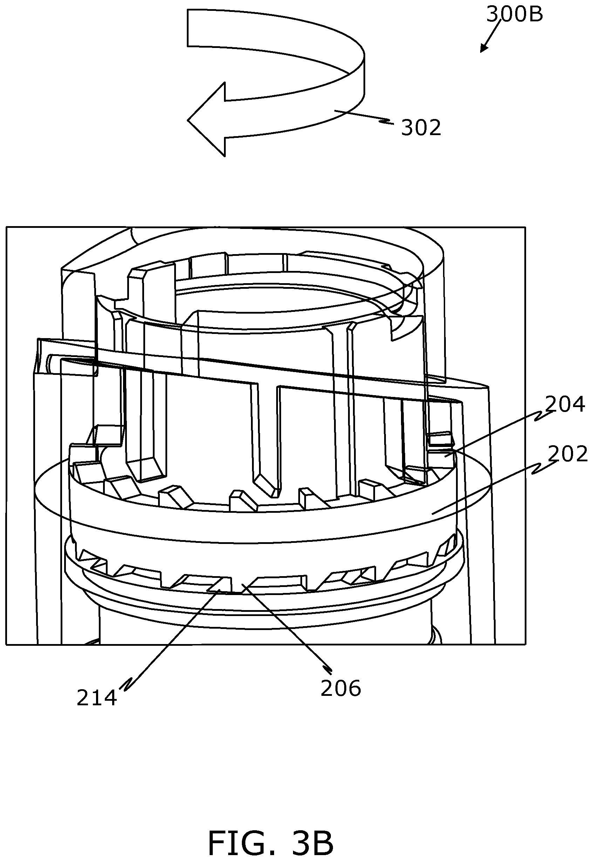

Referring to FIG. 3B, illustrated is a schematic representation a ratchet mechanism **300B** (such as the ratchet mechanism of FIG. 2) in a secondary position of the ratchet ring **202,** in accordance with an embodiment of the present disclosure. When the torque applied to the portable inhaler in the direction of rotation (not shown) is reduced to zero prior to achieving a rotational displacement of 180 degrees, the ratchet ring **202** is retracted back in the clockwise direction **302.** Furthermore, the retraction of the ratchet ring **202** is opposed by the fourth set of teeth **214.** The trailing edges of the teeth of the second set of teeth **206** engage with the leading edges of the teeth of the fourth set of teeth **214,** thereby retaining the position of the ratchet ring **202** that was attained by the application of torque in the direction of rotation. This position of the ratchet ring **202** when the second set of teeth **206** engage with the fourth set of teeth **214** to restrict the movement of the ratchet ring **202** in the direction **302** is the secondary position of the ratchet ring **202.**

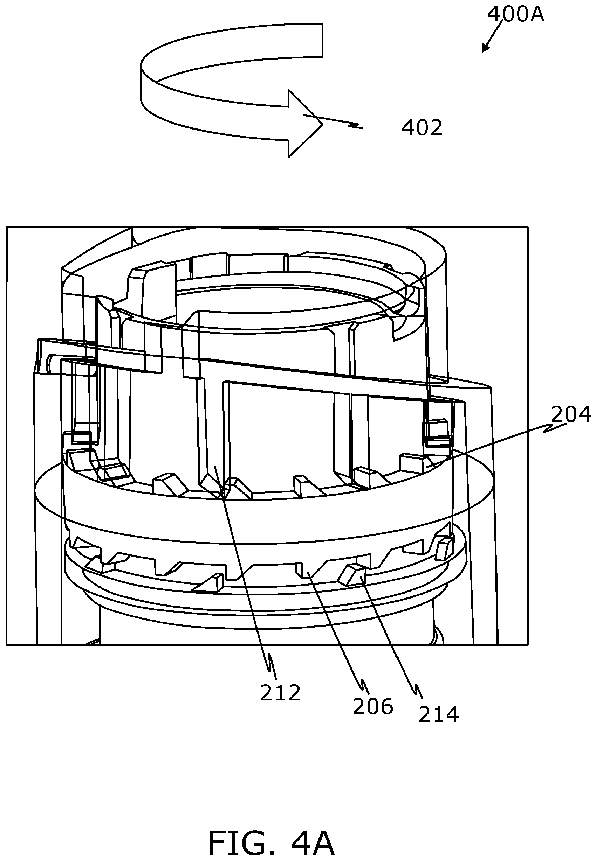

Referring to FIG. 4A, illustrated is a schematic representation a ratchet mechanism **400A** (such as the ratchet mechanism of FIG. 2) in a primary position of the ratchet ring **202,** in accordance with an embodiment of the present disclosure. The trailing edges of the teeth of the first set of teeth **204** is engaged with the leading edges of the teeth of the third set of teeth **212** and the driving component is at the highest position. When the portable inhaler is rotated in the direction of rotation **402** (counter clockwise), each of the leading edges of the teeth of the first set of teeth **204** and the trailing edges of the teeth of third set of teeth **212** interact with each other, and the ratchet ring **202** moves gradually in an axially downward direction and rotates with the driving component as the portable inhaler is rotated.

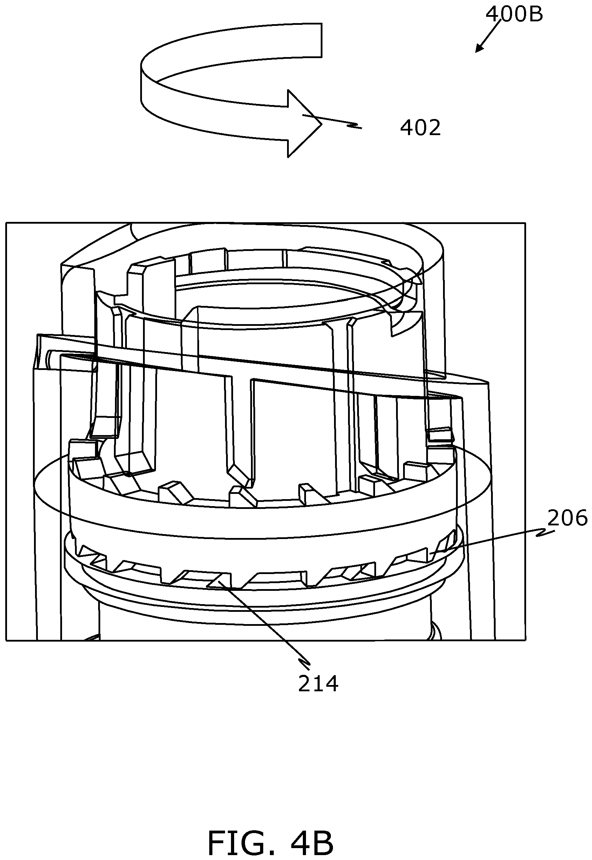

Referring to FIG. 4B, illustrated is a schematic representation of a ratchet mechanism **400B** (such as the ratchet mechanism of FIG. 2) in a secondary position of the ratchet ring, in accordance with an embodiment of the present disclosure. The trailing edges of the teeth of the second set of teeth **206** is engaged with the leading edges of the teeth of the fourth set of teeth **214** and the driving component is at the lowest position. When the portable inhaler is rotated in the direction of rotation **402** (counter clockwise), each of the leading edges of the teeth of the second set of teeth **206** and the trailing edges of the teeth of fourth set of teeth **214** interact with each other, and the ratchet ring **202** moves gradually in an axially upward direction and rotates with the driving component as the portable inhaler is rotated.

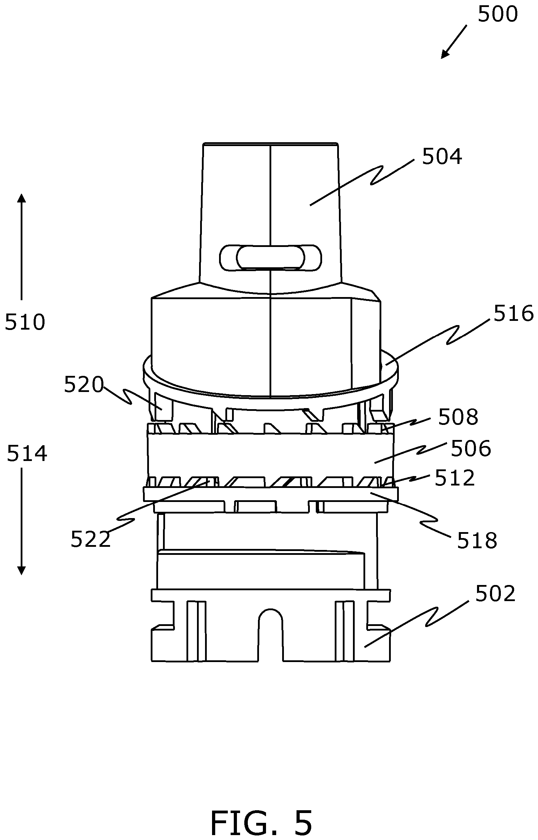

Referring to FIG. 5, illustrated is a schematic representation of a front view of a portable inhaler **500** with the outer casing removed, implementing the ratchet mechanism of FIG. 2, in accordance with an embodiment of the present disclosure. As shown, the portable inhaler **500** comprises a loading end **502** and a mouthpiece **504.** Further, the portable inhaler comprises a ratchet ring **506** having a first set of teeth **508** protruding in a first axial direction **510** and a second set of teeth **512** protruding in a second axial direction **514.** The ratchet ring **506** is arranged between the two annular rings **516** and **518.** The annular ring **516** has a third set of teeth **520** protruding in the second axial direction **514,** and the annular ring **518** has a fourth set of teeth **522** protruding in the first axial direction **510.**

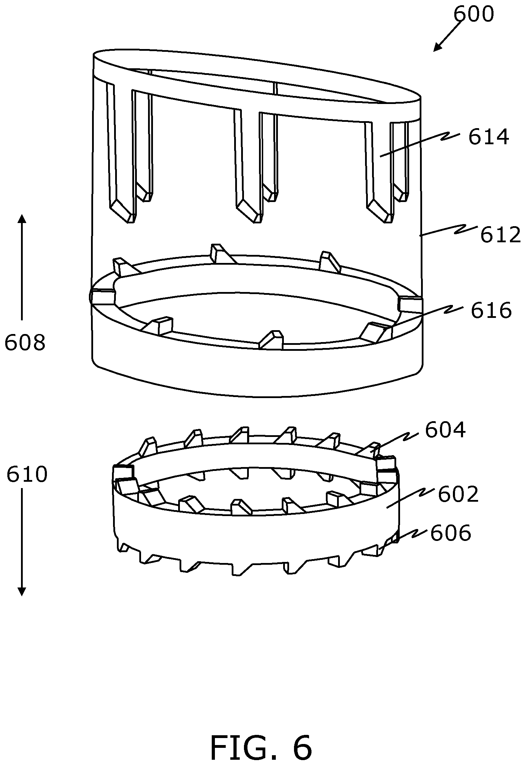

Referring to FIG. 6, illustrated is a schematic representation of an exploded view of the ratchet mechanism **600** (such as the ratchet mechanism of FIG. 2), in accordance with an embodiment of the present disclosure. As shown, the ratchet mechanism **600** comprises a ratchet ring **602** having a first set of teeth **604** and a second set of teeth **606,** each set protruding from opposite surfaces of the ratchet ring **602** in two opposite axial directions, such as the first axial direction **608** and a second axial direction **610.** Further, the ratchet mechanism **600** comprises a chassis **612** having a third set of teeth **614** and a fourth set of teeth **616.** Notably, the ratchet ring **602** is rotationally coupled to a cage component (not shown) and the chassis **612** encloses the ratchet ring **602** coupled to the cage component such that the ratchet ring **602** is snugly fitted between the third set of teeth **614** and the fourth set of teeth **616** of the chassis **612.**

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A portable inhaler (100) comprising a ratchet mechanism, the ratchet mechanism (200) comprising:
- a ratchet ring (202) rotatable about an axis relative to a chassis, with a general direction of rotation determined by teeth of the ratchet mechanism, wherein the ratchet ring has a first set of teeth (204) arranged regularly around the ratchet ring and protruding in a first axial direction from the ratchet ring and a second set of teeth (206) arranged regularly around the ratchet ring and protruding in a second opposite axial direction from the ratchet ring; and
- the chassis which has a third set of teeth (212) arranged regularly and protruding in the second axial direction and generally opposed to the first set of teeth, and a fourth set of teeth (214) arranged regularly and protruding in the first axial direction and generally opposed to the second set of teeth (206)
wherein:
- each tooth protruding in the first axial direction or the second axial direction has a leading edge and a trailing edge with respect to the general direction of rotation of the ratchet ring,
- the trailing edge on each tooth of the first set of the teeth (204) and the second set of teeth (206) is oriented in the axial direction of protrusion and the leading edge on each tooth of the first set of teeth (204) and the second set of teeth (206) is angled towards the trailing edge with respect to the axial direction of protrusion, and
- the leading edge on each tooth of the third set of teeth (212) and the fourth set of teeth (214) is oriented in the axial direction of protrusion and the trailing edge on each tooth of the third set of teeth (212) and the fourth set of teeth (214) is angled towards the leading edge with respect to the axial direction of protrusion.

2. A portable inhaler according to claim 1, wherein the teeth from the first set of teeth and the third set of teeth are arranged to align with each other at different relative position of the ratchet ring and the chassis to those at which teeth from the second set of teeth and the fourth set of teeth are arranged to align.

3. A portable inhaler according to claim 2, wherein the teeth are arranged such that opposition of teeth from the first set of teeth and the third set of teeth occurs at primary positions of the ratchet ring and opposition of teeth from the second set of teeth and the fourth set of teeth occurs at secondary positions of the ratchet ring, such that the primary positions and the secondary positions alternate as the ratchet ring is rotated in the general direction of rotation.

4. A portable inhaler according to any preceding claim, wherein the spacing between distal ends of the third set of teeth and the fourth set of teeth is less than the spacing between distal ends of the first set of teeth and second set of teeth.

5. A portable inhaler according to any preceding claim, wherein the spacing between distal ends of the third set of teeth and the fourth set of teeth is greater than an axial depth of the ratchet ring.

6. A portable inhaler according to any preceding claim, wherein the first set of teeth and the second set of teeth protrude from opposite surfaces of the ratchet ring.

7. A portable inhaler according to any preceding claim, wherein the number of teeth in the first set of teeth and the number of teeth in the third set of teeth are related by an integer multiple.

8. A portable inhaler according to any preceding claim, wherein the number of teeth in the second set of teeth and the number of teeth in the fourth set of teeth are related by an integer multiple.

9. A portable inhaler according to any preceding claim, wherein the number of teeth in the first set of teeth is equal to the number of teeth in the second set of teeth.

10. A portable inhaler according to any preceding claim, wherein the portable inhaler comprises a rotary actuation mechanism having a driving component coupled to rotary movement by a user of a first part of the portable inhaler relative to a second part of the portable inhaler.

11. A portable inhaler according to claim 10, wherein the ratchet ring and the driving component of the rotary actuation mechanism have mutually interfering ribs or slots such that they are rotationally coupled.

12. A portable inhaler according to claim 11, wherein the chassis is the second part of the portable inhaler, wherein the third set of teeth and the fourth set of teeth extend from an inner wall of the chassis.

## Patentansprüche

1. Tragbarer Inhalator (100), umfassend einen Sperrklinkenmechanismus, der Sperrklinkenmechanismus (200) umfassend:
- einen Sperrklinkenring (202), der um eine Achse relativ zu einem Gehäuse drehbar ist, wobei eine allgemeine Drehrichtung durch Zähne des Sperrklinkenmechanismus festgelegt ist, wobei der Sperrklinkenring einen ersten Satz Zähne (204), die regelmäßig um den Sperrklinkenring angeordnet sind und in einer ersten axialen Richtung von dem Sperrklinkenring vorstehen, und einen zweiten Satz Zähne (206), die regelmäßig um den Sperrklinkenring angeordnet sind und in einer zweiten axialen Richtung von dem Sperrklinkenring vorstehen, aufweist; und
- das Gehäuse, das einen dritten Satz Zähne (212), die regelmäßig angeordnet sind und in der zweiten axialen Richtung vorstehen und im Allgemeinen dem ersten Satz von Zähnen gegenüberliegen, und einen vierten Satz Zähne (214), die regelmäßig angeordnet sind und in der ersten axialen Richtung vorstehen und im Allgemeinen dem zweiten Satz von Zähnen gegenüberliegen (206), aufweist,
wobei:
- jeder in der ersten axialen Richtung oder der zweiten axialen Richtung hervorstehende Zahn eine Vorderkante und eine Hinterkante in Bezug auf die allgemeine Drehrichtung des Sperrklinkenrings aufweist,
- die Hinterkante an jedem Zahn des ersten Satzes Zähne (204) und des zweiten Satzes Zähne (206) in der axialen Richtung des Vorsprungs ausgerichtet ist und die Vorderkante an jedem Zahn des ersten Satzes Zähne (204) und des zweiten Satzes Zähne (206) in Bezug auf die axiale Richtung des Vorsprungs zur Hinterkante hin abgewinkelt ist, und
- die Vorderkante an jedem Zahn des dritten Satzes Zähne (212) und des vierten Satzes Zähne (214) in der axialen Richtung des Vorsprung ausgerichtet ist und die Hinterkante an jedem Zahn des dritten Satzes Zähne (212) und des vierten Satzes Zähne (214) in Bezug auf die axiale Richtung des Vorsprungs zur Vorderkante hin abgewinkelt ist.

2. Tragbarer Inhalator nach Anspruch 1, wobei die Zähne des ersten Satzes Zähne und des dritten Satzes Zähne in einer anderen relativen Position des Sperrklinkenrings und des Gehäuses zueinander ausgerichtet angeordnet sind als die Zähne des zweiten Satzes Zähne und des vierten Satzes Zähne zueinander ausgerichtet angeordnet sind.

3. Tragbarer Inhalator nach Anspruch 2, wobei die Zähne so angeordnet sind, dass eine Gegenüberstellung der Zähne des ersten Satzes Zähne und des dritten Satzes Zähne an primären Positionen des Sperrklinkenrings auftritt und die Gegenüberstellung der Zähne des zweiten Satzes Zähne an sekundären Positionen des Sperrklinkenrings auftritt, sodass die primären Positionen und die sekundären Positionen sich abwechseln, wenn der Sperrklinkenring in der allgemeinen Drehrichtung gedreht wird.

4. Tragbarer Inhalator nach einem der vorhergehenden Ansprüche, wobei der Abstand zwischen distalen Enden des dritten Satzes Zähne und des vierten Satzes Zähne kleiner ist als der Abstand zwischen distalen Enden des ersten Satzes Zähne und des zweiten Satzes Zähne.

5. Tragbarer Inhalator nach einem der vorhergehenden Ansprüche, wobei der Abstand zwischen distalen Enden des dritten Satzes Zähne und des vierten Satzes Zähne größer als eine axiale Tiefe des Sperrklinkenrings ist.

6. Tragbarer Inhalator nach einem der vorhergehenden Ansprüche, wobei der erste Satz Zähne und der zweite Satz Zähne von gegenüberliegenden Oberflächen des Sperrklinkenrings vorstehen.

7. Tragbarer Inhalator nach einem der vorhergehenden Ansprüche, wobei die Anzahl der Zähne in dem ersten Satz Zähne und die Anzahl der Zähne in dem dritten Satz Zähne um ein ganzzahliges Vielfaches in Beziehung stehen.

8. Tragbarer Inhalator nach einem der vorhergehenden Ansprüche, wobei die Anzahl der Zähne in dem zweiten Satz Zähne und die Anzahl der Zähne in dem vierten Satz Zähne um ein ganzzahliges Vielfaches in Beziehung stehen.

9. Tragbarer Inhalator nach einem der vorhergehenden Ansprüche, wobei die Anzahl der Zähne in dem ersten Satz Zähne gleich der Anzahl der Zähne in dem zweiten Satz Zähne ist.

10. Tragbarer Inhalator nach einem der vorhergehenden Ansprüche, wobei der tragbare Inhalator einen Drehaktivierungsmechanismus mit einer Antriebskomponente umfasst, die mit einer Drehbewegung eines ersten Teils des tragbaren Inhalators durch einen Benutzer relativ zu einem zweiten Teil des tragbaren Inhalators gekoppelt ist.

11. Tragbarer Inhalator nach Anspruch 10, wobei der Sperrklinkenring und die Antriebskomponente des Drehaktivierungsmechanismus gegenseitig ineinandergreifende Rippen oder Schlitze aufweisen, sodass sie drehbar gekoppelt sind.

12. Tragbarer Inhalator nach Anspruch 11, wobei das Gehäuse der zweite Teil des tragbaren Inhalators ist, wobei sich der dritte Satz Zähne und der vierte Satz Zähne von einer Innenwand des Gehäuses aus erstrecken.

## Revendications

1. Inhalateur portable (100) comprenant un mécanisme à cliquet, le mécanisme à cliquet (200) comprenant :
- un anneau à cliquet (202) pouvant tourner autour d'un axe par rapport à un châssis, avec une direction de rotation générale déterminée par des dents du mécanisme à cliquet, dans lequel l'anneau à cliquet a un premier ensemble de dents (204) agencées de manière régulière autour de l'anneau à cliquet et faisant saillie dans une première direction axiale à partir de l'anneau à cliquet et un deuxième ensemble de dents (206) agencées de manière régulière autour de l'anneau à cliquet et faisant saillie dans une deuxième direction axiale opposée à partir de l'anneau à cliquet ; et
- le châssis qui a un troisième ensemble de dents (212) agencées de manière régulière et faisant saillie dans la deuxième direction axiale et généralement opposé au premier ensemble de dents, et un quatrième ensemble de dents (214) agencées de manière régulière et faisant saillie dans la première direction axiale et généralement opposé au deuxième ensemble de dents (206)
dans lequel :
- chaque dent faisant saillie dans la première direction axiale ou la deuxième direction axiale a un bord d'attaque et un bord de fuite par rapport à la direction générale de rotation de l'anneau à cliquet,
- le bord de fuite sur chaque dent du premier ensemble de dents (204) et le deuxième ensemble de dents (206) est orienté dans la direction axiale de la saillie et le bord d'attaque sur chaque dent du premier ensemble de dents (204) et le deuxième ensemble de dents (206) est incliné vers le bord de fuite par rapport à la direction axiale de la saillie, et
- le bord d'attaque sur chaque dent du troisième ensemble de dents (212) et le quatrième ensemble de dents (214) est orienté dans la direction axiale de saillie et le bord de fuite sur chaque dent du troisième ensemble de dents (212) et le quatrième ensemble de dents (214) est incliné vers le bord d'attaque par rapport à la direction axiale de saillie.

2. Inhalateur portable selon la revendication 1, dans lequel les dents du premier ensemble de dents et du troisième ensemble de dents sont agencées pour s'aligner les unes avec les autres à des positions relatives différentes de l'anneau à cliquet et du châssis par rapport à celles auxquelles les dents du deuxième ensemble de dents et du quatrième ensemble de dents sont agencées pour s'aligner.

3. Inhalateur portable selon la revendication 2, dans lequel les dents sont agencées de telle sorte que l'opposition des dents du premier ensemble de dents et du troisième ensemble de dents se produise au niveau de positions primaires de l'anneau à cliquet et l'opposition des dents du deuxième ensemble de dents et du quatrième ensemble de dents se produise au niveau de positions secondaires de l'anneau à cliquet, de telle sorte que les positions primaires et les positions secondaires s'alternent lorsque l'anneau à cliquet tourne dans la direction générale de rotation.

4. Inhalateur portable selon l'une quelconque des revendications précédentes, dans lequel l'espacement entre les extrémités distales du troisième ensemble de dents et du quatrième ensemble de dents est inférieur à l'espacement entre les extrémités distales du premier ensemble de dents et du deuxième ensemble de dents.

5. Inhalateur portable selon l'une quelconque des revendications précédentes, dans lequel l'espacement entre les extrémités distales du troisième ensemble de dents et du quatrième ensemble de dents est supérieur à une profondeur axiale de l'anneau à cliquet.

6. Inhalateur portable selon l'une quelconque des revendications précédentes, dans lequel le premier ensemble de dents et le deuxième ensemble de dents font saillie à partir des surfaces opposées de l'anneau à cliquet.

7. Inhalateur portable selon l'une quelconque des revendications précédentes, dans lequel le nombre de dents dans le premier ensemble de dents et le nombre de dents dans le troisième ensemble de dents sont liés par un multiple entier.

8. Inhalateur portable selon l'une quelconque des revendications précédentes, dans lequel le nombre de dents dans le deuxième ensemble de dents et le nombre de dents dans le quatrième ensemble de dents sont liés par un multiple entier.

9. Inhalateur portable selon l'une quelconque des revendications précédentes, dans lequel le nombre de dents dans le premier ensemble de dents est égal au nombre de dents dans le deuxième ensemble de dents.

10. Inhalateur portable selon l'une quelconque des revendications précédentes, dans lequel l'inhalateur portable comprend un mécanisme d'actionnement rotatif ayant un composant d'entraînement couplé à un mouvement rotatif par un utilisateur d'une première partie de l'inhalateur portable par rapport à une deuxième partie de l'inhalateur portable.

11. Inhalateur portable selon la revendication 10, dans lequel l'anneau à cliquet et le composant d'entraînement du mécanisme d'actionnement rotatif ont des nervures ou des fentes interférant mutuellement de telle sorte qu'ils sont couplés en rotation.

12. Inhalateur portable selon la revendication 11, dans lequel le châssis est la deuxième partie de l'inhalateur portable, dans lequel le troisième ensemble de dents et le quatrième ensemble de dents s'étendent depuis une paroi intérieure du châssis.
